(19) Europäisches Patentamt
European Patent Office
Office européen des brevets

(11) Publication number: **0 019 937 B1**

(12) # EUROPEAN PATENT SPECIFICATION

(45) Date of publication of patent specification: **05.02.86**

(51) Int. Cl.$^4$: **C 12 N 9/04,** C 12 Q 1/26

(21) Application number: **80103097.4**

(22) Date of filing: **03.06.80**

(54) **A method for producing alcohol oxidase, alcohol oxidase, enzyme preparation and a method for determining the concentration of a compound in an alcohol-containing sample.**

(30) Priority: **05.06.79 US 45715**

(43) Date of publication of application:
**10.12.80 Bulletin 80/25**

(45) Publication of the grant of the patent:
**05.02.86 Bulletin 86/06**

(84) Designated Contracting States:
**DE FR GB IT SE**

(56) References cited:
**GB-A-1 507 810**

**CHEMICAL ABSTRACTS, vol. 89, no. 13, 25th September 1978, page 396, no. 103375t, Columbus, Ohio, U.S.A., YA.A. SIMISKER et al.: "Enzymes participating in the oxidation of methanol in methanol-assimilating yeasts"**

**CHEMICAL ABSTRACTS, vol. 83, no. 1, 7th July 1975, page 1618, no. 1614d, Columbus, Ohio, U.S.A., M. NANJO et al.: "Amperometric determination of alcohols, aldehydes and carboxylic acids with an immobilized alcohol oxidase enzyme electrode"**

(73) Proprietor: **PHILLIPS PETROLEUM COMPANY
5th and Keeler
Bartlesville Oklahoma 74004 (US)**

(72) Inventor: **Hopkins, Thomas R.
3417 S.E. Willowood Drive
Bartlesville Oklahoma 74004 (US)**

(74) Representative: **Dost, Wolfgang, Dr.rer.nat., Dipl.-Chem. et al
Patent- und Rechtsanwälte Bardehle-Pagenberg-Dost-Altenburg & Partner Postfach 86 06 20
D-8000 München 86 (DE)**

(56) References cited:
**CHEMICAL ABSTRACTS, vol. 77, no. 19, 6th November 1972, page 79, no. 122667h, Columbus, Ohio, USA, L.C. CLARK Jr.: "Family of polaro-graphic enzyme electrode and the measurement of alcohol"**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

(56) References cited:

MICROBIOLOGY ABSTRACTS, Section A, INDUSTRIAL AND APPLIED MICROBIOLOGY, vol. 11, no. 9, September 1976, abstract no. 6003, London, G.B., N. KATO et al.: "Alcohol oxidases of Kloeckera sp and Hansenula polymorpha. Catalytic properties and subunit structures"

AGRICULTURAL BIOLOGICAL CHEMISTRY, vol. 43, no. 4, April 1979, pages 877-878, Tokyo, JP., Y. YAMADA et al.: "Purification and characterization of alcohol oxidase from candida 25-A"

Agricultural Biological Chemistry, vol. 38, no. 3, March 1974, pages 675-677

## Description

Field of the invention

The invention relates to a novel alcohol oxidase. In another aspect, the invention relates to a novel crystalline alcohol oxidase. In other aspects, the invention relates to methods for production and isolation of such novel alcohol oxidases.

Background of the invention

Alcohol oxidases are known to be produced by various microorganisms grown on methanol, though not on ethanol. These alcohol oxidases catalyze the reaction

$$RCH_2OH+O_2 \rightleftharpoons RCHO+H_2O_2$$

where R is hydrogen or a lower alkyl, generally selected from the group H—, $CH_3$—, $CH_3CH_2$—, and $CH_3(CH_2)_2$—. Alcohol oxidases can be used to scavenge or remove oxygen from compatible solutions, as well as in the production of aldehydes and hydrogen peroxide. In combination with a suitable test probe, the alcohol oxidase enzyme can be employed to determine alcohol concentration, especially the concentrations of such alcohols as methanol and ethanol. Hence, such enzymes are useful in applications such as the measurement of alcohol levels in biological fluids, for example blood, and the like.

Some uses of these enzymes have been hindered by unavailability of the pure enzyme in commercially reasonable quantities for proposed uses. One of the problems encountered with previous types of alcohol oxidase is that, in common with many enzymes, they are difficult to isolate in relatively pure form, for example in a form essentially lacking catalase activity. A variety of techniques, such as fractional precipitation using materials such as ammonium sulfate, alcohol or polyethylene glycol, or column chromatography using ion exchange resins or gel molecular sieve media, have been utilized to prepare the purified enzyme. Isolation of relatively pure alcohol oxidase by the use of such techniques is difficult, and material and time consuming, accounting for much of the cost of the commercial enzyme. The resulting high costs of relatively pure alcohol oxidases lead to restricted usage of these enzymes, and discourage potential use of the enzyme in application requiring large quantities of the enzyme as well as discouraging the search for new applications. Moreover, as is known in the art, stability of the known prior alcohol oxidase enzymes to temperature and pH, the specificity for particular substrates, and susceptibility to inhibition by these compounds and others, as well as the rate of the catalyzed reaction also affect the potential uses to which such enzymes are most efficiently and effectively placed.

In Agr. Biol. Chem. *38* (1974) 675—677 there is disclosed an alcohol oxidase obtained from microorganisms of Pichia pinus and Pichia trehalophila by way of dialysis of a cell extract according to the method described in Agr. Biol. Chem. *38* (1974) 111—116.

The European Journal of Biochemistry *64* (1976) 341—350 deals with alcohol oxidases of genus' Kloeckera and Hansenula utilizing the method described in Agr. Bio. Chem. *36* (1972) 76—83 and 68—75 and comprising, i.a., an ammonium sulfate fractionation.

Agr. Biol. Chem. *43* (1979) 877—878 describes a method for obtaining crystalline material of Candida genus utilizing a gel filtration with ammonium sulfate precipitation.

Chemical Abstracts *89* (1978) 396, 103375t refers to an alcohol oxidase contained in cell-free extracts of Candida boidinii and Pichia pinus.

Chemical Abstracts *83* (1975) 1618, 1614d mentions the measurement of blood alcohol utilizing immobilized alcohol oxidase without making reference to any species.

Chemical Abstracts *77* (1977) 79, 122667h reports on an experiment using a polarograph for the measurement of alcohol using an alcohol oxidoreductase.

GB—A—1 507 810 relates to a method of measuring the level of ethanol in a body fluid by oxidizing the ethanol by use of an alcohol oxidase. The only alcohol oxidase mentioned is that isolated from a Kloeckera yeast (claim 3 and page 2, line 26).

Summary of the invention

We have now discovered a novel alcohol oxidase exhibiting properties differing from known alcohol oxidases in some of its properties and particularly in its unusual character in ease of crystallizing in recovery operations such as dialysis. The novel alcohol oxidase is obtained from methanol-utilizing *Pichia*-type microorganisms comprising microorganisms of genus *Pichia* and microorganisms genetically and/or taxonomically closely related to *Pichia*.

Accordingly, the invention relates to a method for producing alcohol oxidase from aqueous fluids containing a suspension of cells of a methanol utilizing microorganism of genus Pichia which method is characterized by preparing an aqueous fluid having a cell density effective for crystallization of alcohol oxidase in a recovery range solution having a molar ionic strength in the range of 0.05 M to 0.01 M, homogenizing said cell-containing fluid to produce a homogenate, and then removing suspended solids from said homogenate, thereby producing a solution containing soluble alcohol oxidase.

For obtaining crystalline alcohol oxidase the aforesaid method is further characterized by dialyzing the thus prepared solution across a membrane impermeable to the alcohol oxidase but permeable to water

and buffer molecules, if any, against a dialysis medium to achieve said recovery range solution on an enzyme side of said membrane thereby resulting in crystalline alcohol oxidase, and separating the thus produced crystalline alcohol oxidase from the dialysis medium.

Furthermore, the invention relates to an enzyme preparation from Pichia pastoris catalyizing the following reaction:

$$RCH_2OH+O_2 \rightleftharpoons RCHO+H_2O_2$$

wherein R is hydrogen, methyl, ethyl or propyl, the enzyme being characterized by its capacity to crystallize as an electrophoretically homogeneous crystalline oxidase from an aqueous solution having an effective cell density of at least 75 g (on a dry weight basis) per liter of fluid under dialysis at a pH in the range of 5.75 to 6.75 at a recovery range solution ionic strength from 0.05 to 0.01 molar.

Furthermore, the invention relates to a method for determining the concentration of a compound in a fluid sample containing an alcohol selected from methanol, ethanol, n-propanol and n-butanol which method is characterized by preparing said fluid sample to have conditions compatible with enzymatic activity, adding an alcohol oxidase preparation from a methanol utilizing microorganism of Pichia pastoris to said sample, and measuring a change of concentration in said sample of a compound selected from oxygen, hydrogen peroxide, and product aldehydes.

Furthermore, the invention relates to the alcohol oxidase from Pichia pastoris including the concentrated alcohol oxidase and the crystalline alcohol oxidase from Pichia pastoris.

Finally, the invention relates to an enzyme electrode characterized by a polarographic dissolved oxygen electrode and an alcohol oxidase as described hereinbefore immobilized on the tip of said polarograhic dissolved oxygen electrode.

The crystalline form of the alcohol oxidase is particularly desirable for commerical usages. The crystalline form can be obtained by methods of ultra filtration, presently preferably and conveniently by dialysis; or by other separation methods.

Description of the preferred embodiments

Fermentation process

The alcohol oxidase of the invention is produced by species of *Pichia*-type yeasts which are yeasts of the genus *Pichia* and those which are genetically and/or taxonomically closely related to *Pichia*, preferably of the genus *Pichia* itself, and which are capable of utilizing a feedstock containing methanol as carbon and energy source.

Specific examples of such methanol utilizing *Pichia* yeasts include

*Pichia pastoris*
*Pichia pinus*
*Pichia trehalophila*
*Pichia molischiana*

Two exemplary strains of suitable yeasts of the species *Pichia pastoris* have been deposited with the United States Department of Agriculture, Agriculture Research Service, Northern Regional Research Laboratories of Peoria, Illinois, and have received the numerical designations NRRL Y-11430 and Y-11431.

According to the present invention, a selected species of methanol competent *Pichia*-type yeast is cultured under aerobic aqueous fermentation conditions using methanol as the carbon and energy source. Preferably the methanol is supplied under conditions so that methanol is the growth-limiting factor. The methanol limiting conditions are defined for purposes of the disclosure as a concentration of methanol which is the minimal concentration of methanol which results in a maximum growth rate for a given set of fermentation culture conditions. Preferably fermentation is conducted under high cell density conditions, *i.e.*, so that cell density is 100 grams or greater on a dry weight basis per liter of ferment. The selected yeast is grown in a batch or continuous process in the presence of oxygen, methanol, and an assimilable source of nitrogen. Various types of fermentation processes and apparatuses known in the art can be utilized. For example, a foam-type fermenter such as described in US—A—3,982,998, or other suitable fermenter can be used.

Oxygen can be supplied to the fermenter as such, or in the form of air or oxygen-enriched air, in a range of pressures from such as 0.1 atm. to 100 atm. (0.01—10 MPa), as is known in the art. The assimilable source of nitrogen for the fermentation can be any organic or inorganic nitrogen containing compound which provides nitrogen in a form suitable for metabolic utilization by the microorganisms. Suitable organic nitrogen sources include, for example, proteins, amino acids and urea. Suitable inorganic nitrogen sources include, for example, ammonia, ammonium hydroxide and ammonium nitrate. The presently preferred nitrogen sources include ammonia and ammonium hydroxide for convenience and availability.

The pH range in the aqueous microbial ferment should be in the range of 3 to 7, more preferably and usually 3.5 to 5.5. Preferences of certain microorganisms for a pH range are dependent to some extent on the medium employed, as well as on the particular microorganism, and thus may change somewhat with change in medium as can be readily determined by those skilled in the art.

Sufficient water is maintained in the fermentation means so as to provide for the particular requirements of the microorganism employed as well as to provide a carrier fluid for water soluble

nutrients. Minerals, growth factors, vitamins, and the like, generally are added in amounts which vary according to the strain of microorganism utilized and the selected culture conditions, and are known to those skilled in the art or are readily determinable by them. A typical nutrient medium is set forth below in the introduction to the examples.

The growth of the microorganism is sensitive to the operating temperature of the fermenter and each particular strain of microorganism has an optimum temperature for growth. Exemplary fermentation temperatures are in the range of 20 to 65°C. The temperature selected will generally depend upon the microorganism employed in the process since each one will have a somewhat different temperature/growth rate relationship.

Fermentation pressures are generally within the range of 0.1 to 100 atmospheres (0.01—10 MPa) more usually 1 to 30 atmospheres (0.1—3 MPa) and more preferably 1 to 5 atmospheres (0.1—0.5 MPa) since the higher pressures results in a greater level of dissolved oxygen in the aqueous medium and usually higher cell productivities.

Alcohol oxidase isolation

In the isolation of the novel alcohol oxidase a fluid is prepared which is an aqueous suspension containing cells of the selected microorganism. The aqueous fluid can be fermenter effluent which can be used directly, or preferably after adjusting the pH as described below. Alternatively the suspended microorganism cells can be initially separated from the fermentation medium, for example, by centrifugation or by filtration through filters having a pore size less than the size of the individual cells, and subsequently resuspended in a convenient volume of water or of an appropriate aqueous buffer, for example $KH_2PO_4/Na_2HPO_4$ buffer at 0.2 M. It has been found that the cell density in the aqueous suspension must be greater than a minimum crystallization density. Satisfactory results are obtained if the fluid cell density is greater than about 75 grams on a dry weight basis per liter of fluids. It has been found that satisfactory results are obtained if the fermenter effluent, where it is to be used as the fluid, is first adjusted to a pH of such as about 7.5 by addition of a base such as ammonium hydroxide or sodium hydroxide. The pH is not considered critical, however and the pH of the aqueous suspension need not be adjusted prior to homogenization. However, it is considered preferable to adjust the pH broadly in the range of 6—9 since in this range the enzyme is active and stable.

The cell-containing fluid is homogenized by suitable means known in the art. For example, fermenter effluent containing yeast grown on methanol can be adjusted to a pH of about 7.5 and homogenized at a high cell density concentration such as 100—120 grams biomass (dry weight)/liter using a Dynomill(TM) Model KDL using a 0.6 liter vessel in a continuous operation at 5 to 30°C using belt combination #3 and a flow of 20—30 ml/hr. The homogenate solids are separated from the homogenate to produce a crude solution containing the novel alcohol oxidase as a soluble component. For example, the homogenate solids can be removed by centrifugation to yield a cell-free supernatant. Alternatively the solids can be removed by filtration through filters having a suitable pore size, followed by pH adjustment if desired. If desired, for further purification steps such as recovery of crystalline alcohol oxidase, the pH can be adjusted to have a pH in the range of 5.75 to 6.75 as desired, for example, to pH 6.5.

The crude solution containing my alcohol oxidase has effective enzymatic activity and finds useful applications in that form. As such, it constitutes a part of my invention. However, the alcohol oxidase of my invention has specific properties as hereinafter set forth which are best realized by the isolation of the crystalline alcohol oxidase.

Preparation of crystalline alcohol oxidase

The crude solution containing the soluble alcohol oxidase can be treated to recover the novel alcohol oxidase either in more concentrated solid form by such as by fractional precipitation with ammonium sulfate, or most desirably and preferably as the potent crystalline form exhibiting highest activity by treatment under dialysis conditions either by conventional dialysis modes or by applying ultrafiltration to increase the rate of recovery.

In dialysis, the crude solution containing the soluble alcohol oxidase is dialyzed against a dialysis medium across a membrane impermeable to alcohol oxidase but permeable to water, buffer, and inorganic molecules. The crude solution is prepared by homogenizing an aqueous fluid having a cell density effective for crystallization of alcohol oxidase when the solution attains a recovery range solution condition as herein described. Satisfactory crystallization has been observed where the effective cell density is about 75 grams (on a dry weight basis) per liter of aqueous fluid. Crystallization is also expected to occur at even lower effective cell densities although the amount of crystalline alcohol oxidase recovered is less. Below an empirically determinable minimum cell density (minimum crystallization density) essentially no crystalline alcohol oxidase is recovered. The type of membrane used is not considered critical and any suitable membrane may be used. For example, commercially available cellulose acetate dialysis tubing can be used to form dialysis bags or otherwise used, or hollow fiber dialysis cells can be used. The alcohol oxidase containing solution is dialyzed against a dialysis medium, for example water or a buffer solution, to achieve a recovery range solution on the enzyme side of the membrane having an ionic strength in a recovery range of between 0.05 M and 0.01 M thereby effecting precipitation of an electrophoretically homogeneous crystalline oxidase.

The dialysis medium can be any medium whereby during dialysis the molar ionic strength of the solution on the enzyme side of the membrane passes through at least a portion of the recovery range. For example, if the crude solution containing alcohol oxidase has a molar ionic strength of 0.2 M, the dialysis medium can be a suitable volume of distilled water. The volume of fluid against which the enzyme is dialyzed is not considered critical so long as the ionic strength on the enzyme side of the membrane passes through at least a portion of the recovery range.

During dialysis, the pH of the alcohol oxidase containing solution should be maintained in the range of 5.75 to 6.75 by use of a suitable buffer system. A suitable buffer system comprises, for example, potassium dihydrogen phosphate and disodium hydrogen phosphate. Preferably the pH range is from 6.0 to 6.5 for recovery of maximum amounts of crystalline alcohol oxidase. As shown in the example below, good crystallization of the alcohol oxidase has been observed within the broad pH range, and the narrow range represents a presently preferred pH range to achieve minimum solubility of the enzyme.

The alcohol oxidase thus obtained has been found to have minimum solubility under these conditions in solutions of 0.02 M ionic strength at pH 6.0 to 6.25. Consequently, optimum crystallization is achieved by planning the dialysis to obtain these conditions. Good crystallization can be achieved by exhaustive dialysis of the enzyme containing solution against large volumes of buffers meeting the above conditions. Alternatively, the dialysis system can be designed to achieve optimal crystallization conditions either at equilibrium or at a point in time after the start of dialysis. For example, a crude enzyme solution having an ionic strength of 0.2 M at pH 6.25 can be dialyzed against a nine-fold excess of distilled water (relative to the volume of the crude enzyme solution). At equilibration, the ionic strength of the crude enzyme solution will be 0.02 M and crystallization will occur. Such a method has the disadvantage that a relatively long period of time is required for equilibration to occur.

On the other hand, if the crude enzyme solution has a molar ionic strength of, for example, 0.05 M, dialysis against a nine-fold excess of distilled water (relative to the volume of the crude enzyme solution) to equilibration will results in a solution having 0.005 M ionic strength and crystals formed will tend to redissolve since the equilibrium ionic strength is outside the recovery range. However, the crystals will form after a relatively shorter dialysis time and may then be removed and recovered before system equilibration and redissolution. This latter method of dialysis is presently preferred because of the decreased time required to recover crystalline alcohol oxidase.

The dialysis can be safely carried out at temperatures in the range from 4 to 40°C. Sufficient time, generally more than one hour, and preferably 18 hours, or more, must be allowed for crystallization to occur.

At the end of dialysis, the alcohol oxidase is present in the dialysis bag as a crystalline solid. The crystalline alcohol oxidase can be readily separated from the dialysis medium, such as by decanting the liquid in the dialysis bag from the solid crystals. The moist crystals can be further processed as desired for storage. For example, the crystal slurry can be frozen followed by lyophilization to form a dry powder, or can be dissolved in water or more preferably in a phosphate buffer. Stabilizer compounds known to stabilize enzyme solutions against denaturation and loss of enzymatic activity can be added, such as sucrose or glycerol. It is preferable to store the prepared enzyme at temperatures in the range of 4 to 40°C. More preferably, the enzyme is stored at temperatures in the range of 4 to 24°C. Most preferable is storing the enzyme at about 4°C. Only minimal loss of activity has been found to occur when the enzyme is stored at 4°C in 0.1 M phosphate buffer at pH 7.5, and with 0.02% sodium azide to inhibit microorganism growth. However, the alcohol oxidase can also be stored frozen without significant loss of enzymatic activity.

In the process of preparing alcohol oxidase from *Pichia* microorganisms according to the invention, a crystalline solid is formed during dialysis of the crude enzyme solution and no further purification steps have been found necessary. The novel crystalline alcohol oxidase is a readily prepared and relatively inexpensive alcohol oxidase available for applications otherwise economically unattractive.

Characterization of *Pichia* alcohol oxidase

The alcohol oxidase isolated from *Pichia*-type microorganisms is typified by the alcohol oxidase isolated from *Pichia pastoris*. The "Pichia" alcohol oxidase is homogeneous as judged by sodium dodecyl sulfate (SDS) gel electrophoresis. Very little, if any, of the original catalase activity of the cells remains associated with the crystalline alcohol oxidase. The alcohol oxidase enzyme is estimated to comprise 6 or more subunits, of an estimated molecular weight of 72,000 per subunit as estimated by SDS gel electrophoresis and a rough estimate of the molecular weight of the alcohol oxidase. The enzyme is a flavoprotein having FAD (flavin adenine dinucleotide) as a coenzyme comprising about one FAD moiety per enzyme subunit. The apparent Michaelis constant, Km, for methanol is about 4 mM. Electrophoretic analysis suggests that the molecular weight of the *Pichia* enzyme is larger than that of an alcohol oxidase isolated from *Candida boidinii*. The *Pichia* enzyme differs from an alcohol oxidase isolated from *Hansenula polymorpha* in the extent to which it binds sodium azide, and in its ability to form crystals in 0.02 M sodium phosphate at pH 6.5.

Characteristics of the *Pichia* enzyme have been determined and are shown in Table I. Reactivities toward various substrates are shown normalized with reference to methanol which is set equal to 100%.

TABLE I

| Characteristic | Pichia pastoris |
|---|---|
| Molecular wt. | 500,000 (est.) |
| Coenzyme | FAD |
| No. of subunits | 6 or more (est.) |
| Optical Activity | |
| Temperature (°C) | |
| (broadly) | 35°—45°+ |
| (optimum) | 45° |
| pH | |
| (broadly) | 6—9 |
| (optimum) | 8.0 |
| Km for methanol (mM) | 4 |
| Inhibitors | HCHO >30 mM |

The *Pichia* alcohol oxidase differs from other reported alcohol oxidases in a number of ways. In particular, the alcohol oxidase from *Pichia pastoris* is reactive toward the lower alcohols and formaldehyde, but is not reactive toward acetaldehyde or organic acids. This lack of reactivity toward acetaldehyde and organic acids is of distinct benefit, for example in use of the *Pichia*-derived alcohol oxidase in procedures for determining alcohol concentration in organic fluids such as blood because interference due to the presence of aldehydes and organic acid materials is avoided.

Alcohol oxidase electrode

The alcohol oxidase of the invention catalyzes the following reaction

$$RCH_2OH+O_2 \rightarrow RCHO+H_2O_2$$

where R is hydrogen or lower alkyl, generally selected from the group consisting of H—, $CH_3$—, $CH_3CH_2$—, and $CH_3(CH_2)_2$—. Accordingly, the enzyme can be used for the production of aldehydes and hydrogen peroxide as well as for the removal of oxygen from enzyme compatible fluids where the presence of oxygen is undesirable.

Furthermore, since in the course of the reaction oxygen is consumed and aldehydes and hydrogen peroxide are produced, the enzyme can be used to determine the concentrations of short chain alcohols $RCH_2OH$ in a fluid sample under conditions compatible with enzymatic activity. For example, the enzyme can be used to determine the concentration of lower alcohols in biological fluids such as the concentration of methanol in a fermentation process or the concentration of ethanol in body fluids such as blood.

A particularly convenient way of determining such alcohol concentrations is to immobilize the alcohol oxidase on the tip of a polarographic dissolved oxygen electrode. Several such polarographic dissolved oxygen electrodes are commercially available and are suitable for utilization with the alcohol oxidase enzyme. For example, a Clark or a Beckmen dissolved oxygen electrode can be used.

The alcohol oxidase can be immobilized on the electrode tip by any suitable method. For example, the enzyme can be blended with suitable supporting materials to form a paste which is held as a thin film on the electrode tip by a membrane permeable to the compound whose concentration is to be determined, but impermeable to the enzyme itself. For example, the supporting material may be DEAE Sephadex(TM), a polysaccharide ion exchange resin from Pharmacia Fine Chemicals, Sweden. For ethanol determinations, a suitable membrane is cellulose acetate film through which film ethanol has a satisfactory mobility. Of course, the enzyme can also be covalently bonded to an appropriate electrode membrane or can be physically incorporated in an appropriate polymer film.

Conditions compatible with enzymatic activity include a fluid sample pH in the range of 6 to 9 and preferably about pH 8.0 for maximum sensitivity, and a fluid sample temperature in the range of 25°C up to and including 45°C and preferably at about 25°C for convenience. The sample pH can be adjusted with ammonium hydroxide solutions or dilute hydrochloric acid solutions as required.

Preferably, a calibration curve is prepared using a series of known concentrations of the compound to be assayed and the concentration of the compound in the fluid sample is determined therefrom as is known in the art.

To determine the alcohol concentration, the sample electrode is immersed in an aliquot of the thus prepared fluid to be examined. The substrate alcohol diffuses across the membrane and in the presence of oxygen reacts to produce an aldehyde product and hydrogen peroxide. The reaction is followed by observing the rate of change of oxygen concentration in the sample. Since the catalyzed reaction is stoichiometric, the concentration of the alcohol $RCH_2OH$ can be determined from the rate of change in oxygen concentration as is known in the art. Alternatively, the reaction can be followed polarographically with reference to the hydrogen peroxide produced. In yet another application, the reaction can be followed galvimetrically.

The use of the alcohol oxidase for the determination of alcohol concentration in biological fluids, for example, determination of ethanol concentration in a blood sample or short chain alcohol concentration in fermenter broths, is particularly advantageous in view of the relatively low reactivity of the instant oxidase to aldehydes and organic acids and is thus not so subject to interference by such compounds which may be present in biological fluids such as blood.

To further illustrate the instant invention, the following examples are provided.

Examples

The following fermentation is typical of the several fermentations carried out to provide the effluent for isolation of the alcohol oxidase.

In a continuous aerobic fermentation process, methanol and an aqueous mineral salts medium in a volume ratio of about 40 to 60, respectively, were fed individually to a fermenter, innoculated with the yeast species *Pichia pastoris* NRRL Y-11430, at a rate so that methanol is the growth-limiting factor. The fermenter was a 1500-liter foam-filled fermenter with a liquid volume of about 610 liters, with automatic pH, temperature, and level control. Agitation was provided by two conventional paddle-type turbines driven at 1000 rpm. The aeration rate was about 4 volumes of air (at about 38 psig (262 kPa) and about 25°C) per volume of ferment in the fermenter per minute. Anhydrous ammonia was added at such a rate as to maintain the pH of the fermentation mixture at about 3.5.

The aqueous mineral salts medium was prepared by mixing, with each liter of tap water, 15.86 ml 75 percent $H_3PO_4$, 9.53 g $K_2SO_4$, 7.8 g $MgSO_4 \cdot 7H_2O$, 0.6 g $CaCl_2 \cdot 2H_2O$, and 2.6 g 85 percent KOH. The trace mineral solution plus biotin was fed separately via the methanol stream at a rate of 10 ml per liter of methanol. The trace mineral solution plus biotin was prepared by mixing 780 ml of a trace mineral solution, 20 ml water, 200 ml methanol and 0.032 g biotin.

The trace mineral solution was prepared by mixing, for each liter of solution, 65 g $FeSO_4 \cdot 7H_2O$, 20 g $ZnSO_4 \cdot 7H_2O$, 3.0 g $MnSO_4 \cdot H_2O$, 6.0 g $CuSO_4 \cdot 5H_2O$, 5.0 ml conc. $H_2SO_4$, and sufficient deionized water to make 1 liter of solution.

The aqueous mineral salts medium was fed at a rate of 31.5 liters per hour and the methanol at a rate of 21 liters per hour.

The fermentation was conducted at about 30°C and about 38 psig (262 kPa) pressure, with a retention time of 11.6 hours.

For analytical purposes, the resulting yeast cells were separated from the fermentation effluent (ferment) by centrifugation, washed by suspension in water and recentrifugation, dried overnight at 100°C, and weighed. On a dried basis, the yield of yeast cells typically was about 40.6 g per 100 g of methanol fed. The cell density typically was about 128.4 g of cells per liter of fermenter effluent. The total solids content of the ferment typically was about 134.7 g per liter, cells plus dissolved solids. A portion of the fermenter effluent was frozen and stored.

In Examples I, IX, and X, the alcohol oxidase activity for reaction with methanol was determined by the following assay procedure (Procedure A). A dye-buffer mixture was prepared by mixing 0.1 ml of an o-dienisidine solution (1 weight% o-dianisidine in water) with 12 ml of aerated 0.1 M sodium phosphate buffer (pH 7.5). The assay mixture was prepared with 2.5 ml of the dye-buffer mixture, 50 μl of methanol, 10 μl of a peroxidase solution (1 mg of horse-radish peroxidase-Sigma, Type II), and 25 μl of the alcohol oxidase solution. The assay mixture was maintained at 25°C in a 4×1×1 cm cuvette and the increase in absorbance by the dye at 460 nm was recorded for 2 to 4 minutes. The enzyme activity was calculated by

$$\text{Activity } (\mu \text{ mole/min/ml}) = \frac{\Delta A}{\text{min}} \times 11.5$$

wherein 11.5 is a factor based on a standard curve prepared with known aliquots of $H_2O_2$ and ΔA is the change in absorbance during the experimental interval.

In Examples III and IV another assay procedure (Procedure B) was used. A MBTH stock solution was prepared with 0.04 g of MBTH (3-methyl-2-benzothiazoline hydrazone, available from Sigma Chemical Company, St. Louis, Missouri) per 100 ml of a 0.05 M phosphate buffer (pH 7.5). A ferric chloride stock solution was prepared from 0.2 g of ferric chloride per 100 ml of 0.1 N HCl. A 1 ml portion of the MBTH solution was added to 25 μl of the alcohol and they were mixed. A 25 μl solution of the alcohol oxidase solution was added and the mixture was incubated for 10 minutes at 25°C. A 4 ml sample of the ferric chloride solution was added to the mixture and the resulting mixture was allowed to stand for 1 hour at the

desired temperature. The dye absorbance at 625 nm was recorded on a colorimeter using a 1 cm path length cuvette. The sample activity is reported as the absorbance peak height.

Example I

Fermentation of *Pichia pastoris* NRRL Y-11430 was carried out by a method of which that set forth above is typical. A portion of the fermenter effluent was removed and adjusted to pH 7.5 with ammonium hydroxide, and was homogenized on a Dyno-Mill(TM) Model KDL using a 0.6 liter vessel in a continuous operation at 30°C using belt combination #3 and a flow of 20—30 ml/hr. The beads in the mill were lead free glass beads with a diameter of 0.3—0.5 mm. The resulting homogenate was centrifuged at 5°C and 20,000×g for 30 minutes to yield a cell-free supernatant. The cell-free supernatant enzyme activity (using Procedure A) was about 330 U/ml. The supernatant was stored frozen for future use.

Six 130 ml portions of the supernatant were placed in cellulose acetate dialysis bags and dialyzed at 5°C against about 8 liters of distilled water. After 4 days, the aqueous phase of each bag was decanted. The solids remaining in the bags consisted of two types of solid. The thin upper white layer was carefully removed and discarded. The bottom solid was brown-yellow and was the alcohol oxidase. A portion of the alcohol oxidase was dissolved in distilled water (about 10 times the volume of the solid) and an assay by Procedure A showed an activity of 94 U/ml. The specific activity of the alcohol oxidase was 10.4 U/mg of protein.

A sample of the solid alcohol oxidase was examined by SDS(TM) gel electrophoresis and a single band was observed indicating a homogeneously pure enzyme. A comparison of electrophoretic mobility with those of proteins having known molecular weight indicates a subunit molecular weight of about 72,000.

The results of this example demonstrate the process of the invention for the preparation and isolation of pure crystalline alcohol oxidase from *Pichia pastoris.*

Example II

A portion of frozen supernatant that had been prepared as described in Example I was thawed and centrifuged to clarify the solution. Six dialysis bags containing 1 ml of the clarified supernatant were each extensively dialyzed overnight against 500 ml of aqueous solutions containing a phosphate buffer of differing ionic strengths (all at pH 7.5). In dialyses 1, 2 and 3 at 0.5 M, 0.1 M and 0.05 M phosphate respectively, no precipitate was observed. The maximum amount of precipitate was observed in dialysis 4 at 0.02 M phosphate. Dialyses 5 and 6 at 0.01 and 0.005 M phosphate, respectively, contained less precipitate than in dialysis 4. However, in dialyses 5 and 6, some of the precipitate formed earlier had redissolved.

The results of these runs demonstrate that precipitation of the alcohol oxidase during dialysis occurs at phosphate buffer levels below about 0.05 M. The alcohol oxidase appears to be least soluble at 0.02 M phosphate. At buffer levels of about 0.01 M and below, dialysis beyond the time required for maximum precipitation can result in redissolving of the crystalline solids.

Example III

A series of assays using assay Procedure B was carried out using different pH values to determine the relative activities of the alcohol oxidase (as the cell-free supernatant from a homogenation) at various levels of pH. The pH of each assay solution was varied by the addition of HCl or NaOH. The relative activity at each pH is expressed as an absorbance at 625 nm.

| pH of solution | Absorbance at 625 nm |
|---|---|
| 4.1 | 0.06 |
| 5.1 | 0.19 |
| 6.1 | 0.33 |
| 7.1 | 0.38 |
| 8.1 | 0.41 |
| 9.1 | 0.33 |
| 10.3 | 0.09 |

These results indicate that the pH optimum is about pH 8 and a working range is from pH 6 to 9.

Example IV

Another series of assays using assay Procedure B was carried out using different assay temperatures to determine the influence of assay temperature on relative activity. Samples of alcohol oxidase from

*Pichia pastoris* were dissolved in 0.05 M phosphate buffer (pH 7.5). The samples were assayed at various assay temperatures and the relative activities are expressed as an absorbance at 625 nm.

| Assay temperature, °C | Absorbance at 625 nm |
|---|---|
| 27 | 0.42 |
| 36 | 0.54 |
| 45 | 0.60 |
| 55 | 0.27 |
| 65 | 0.05 |

These results demonstrate that the temperature optimum for the alcohol oxidase activity from *Pichia pastoris* is about 45°C and the working range is from 35°C to 45°C inclusive.

Example V

In a control run, the alcohol oxidase from *Hansenula polymorpha* was dialyzed according to the method of the invention to demonstrate that an alcohol oxidase from a yeast genus other than *Pichia* did not form a pure, crystalline solid as does the invention. A continuous aerobic aqueous fermentation was carried out in which a yeast species *Hansenula polymorpha* (NRRL Y-11170) was grown on methanol under aqueous fermentation conditions. A portion of the fermenter effluent was homogenized and centrifuged as described in Example I. The cell-free supernatant was dialyzed at 0°C against a 0.005 M phosphate buffer in a series of dialyses using pH values from 5.5 to 7.75 at 0.25 pH intervals. In each dialysis no precipitate formed in the dialysis bag after 20 hours.

In a series of comparison dialyses carried out at the same time with the alcohol oxidase derived from *Pichia pastoris* NRRL Y-11430, precipitation of the crystalline alcohol oxidase occurred at pH values between 5.75 and 6.75. The largest amounts of precipitate were formed at pH 6.0 and 6.25.

The results of these runs show that the alcohol oxidase from *Hansenula polymorpha* does not crystallize during dialysis under conditions effective for the crystallization of the alcohol oxidase of *Pichia pastoris*. This difference in crystallization behavior is notable. It demonstrates a significant advantage for my alcohol oxidase obtained from the *Pichia pastoris* because of the rapid and inexpensive method of recovery of pure crystalline enzyme.

Example VI

The alcohol oxidase isolated from *Pichia pastoris* was immobilized on the tip of a dissolved oxygen electrode for use in the determination of the amount of ethanol in samples. The solid alcohol oxidase and DEAE Sephadex(TM) (in a weight ratio of about 1:1) were mixed and applied to the Teflon(TM) membrane on the tip of a Beckman(TM) dissolved oxygen probe. A cellulose acetate dialysis membrane was used to hold the alcohol oxidase-DEAE Sephadex(TM) mixture to the electrode tip. The electrode was attached to an analog differentiator which gave the analysis results as a peak height which is proportional to the alcohol concentration in the sample. The electrode was calibrated with a series of standard ethanol solutions. In each case, the sample was added to the electrode chamber which contained a 3.3 ml of 0.05 M phosphate buffer (pH 7.5) at 25°C. The calibration curve of peak height vs. alcohol concentration was linear up to a final concentration at the tip of the alcohol oxidase electrode of about 0.1 volume% alcohol (1000 ppm). The alcohol oxidase electrode as constructed worked effectively for alcohol determinations for at least one month. The electrode exhibited rapid response (assay time of about 15 seconds) and was sensitive to concentrations of ethanol as low as 0.2 ppm by volume.

Example VII

The alcohol oxidase electrode described in Example VI was used to determine the relative reactivity of the alcohol oxidase towards various substrates. In each determination, a 1 to 10 µl solution of the substrate in water (1 volume%) was added to the 3.3 ml phosphate buffer (pH 7.5) in the electrode chamber and the peak height was recorded. The results are listed below with the relative reactivities corrected for sample size and normalized with methanol set equal to 100.

| | Relative reactivity |
|---|---|
| Methanol | 100 |
| Ethanol | 27 |
| 1-Propanol | 10 |
| 1-Butanol | 5 |
| Formaldehyde | 30 |

2-Propanol, 2-methyl-1-propanol, 1-pentanol, acetaldehyde, and ethylene glycol had relative activities less than one. Sodium formate, sodium acetate, cyclohexanol, and 1,4-butanediol were essentially inactive.

The results of this example show that the alcohol oxidase electrode of this invention is highly specific for short chain alcohols and formaldehyde. The differences in reactivities of the enzyme observed in this example and those observed in Example XII are believed due in part to different mobilities of the substrate molecules across the cellulose acetate membrane employed in this run. The differences may also be due in part to differences in purity of the prepared alcohol oxidase.

Example VIII

A series of samples of human blood serum were mixed with known quantities of absolute ethanol, and a series of samples of 0.05 M phosphate buffer were also mixed with known quantities of absolute ethanol. In both series, the ethanol levels were 0, 0.01, 0.02, 0.05, and 0.10 volume% ethanol. 50 µl samples from each solution were assayed using the alcohol oxidase electrode described in Example VI.

Plots of peak heights vs. ethanol levels gave linear and nearly identical plots for both series of samples. These results show that other components, for example, aldehydes and organic acids, in blood serum do not interfere with alcohol concentration determination using an alcohol-sensing electrode containing the alcohol oxidase from a *Pichia*-type microorganism.

Example IX

A series of samples containing alcohol oxidase isolated as the pure crystalline solid by dialysis from *Pichia pastoris* NRRL Y-11430 were held at 40°C for up to two weeks to determine thermal stability. The alcohol oxidase was added to a 0.5 M phosphate buffer (pH 7.5) at a concentration of 2.2 mg protein/ml. Another sample of alcohol oxidase was dissolved in 50 volume% aqueous glycerol. Another sample of alcohol oxidase was lyophilized. The three samples were assayed by Procedure A at intervals during the two-week test. The results are summarized below.

| Alcohol oxidase | Original enzyme activity, U/ml | Enzyme activity, U/ml | | |
|---|---|---|---|---|
| | | 2 Days | 1 Week | 2 Weeks |
| Buffer | 31 | 34 | 31 | 30.5 |
| 50% Glycerol | 29 | 51 | 30 | 18 |
| Dry Solid[a] | 13 | 6 | 3.5 | 2 |

[a] The solid was dissolved in a 0.05 M phosphate buffer (1 mg protein/ml) for each assay.

The results of these runs show that the activity of the alcohol oxidase in the buffer solution was essentially unchanged after two weeks at 40°C. The activity of the alcohol oxidase in 50% glycerol was unchanged after one week, but had lost some of its activity after two weeks at 40°C. The freeze dried solid lost most of its activity during the thermal test. However, the lyophilized alcohol oxidase had excellent stability at 4°C.

Example X

A study was made to determine the stability of the alcohol oxidase derived from a *Pichia pastoris* in solution at several temperatures. The fermenter effluent from the aerobic fermentation of *Pichia pastoris* NRRL Y-11430 was homogenized and centrifuged as described in Example I. The cell-free supernatant was fractionally precipitated with ammonium sulfate. The resulting enzyme was estimated from its enzymatic activity to be less than 50% pure.

The thus partially purified alcohol oxidase (0.1 ml) was added to 10 ml samples of distilled water containing 0.02 weight% sodium azide (hereinafter referred to as sodium azide solution) and to 10 ml samples of 0.1 M phosphate buffer (pH 7.5) containing 0.02 weight% sodium azide (hereinafter referred to

as sodium azide-buffer solution). The solutions were held at 4°, 24°, 30°, or 40°C for one month with intervallic assays of aliquots for activity using Procedure A. The sodium azide and sodium azide-buffer solutions held at 4°C showed no loss in activity during the test period. The sodium azide and sodium azide-buffer solutions held at 24°C were estimated by extrapolation of test data to lose about half of their activity (half-life) in 40 days. At 30°C, the sodium azide-buffer solution had a half-life of about 30 days while the sodium azide solution had a half-life of about 10 days. At 40°C the sodium azide-buffer solution had a half-life of about 15 days, while the sodium azide solution had a half-life of only about 3 days.

Example XI

A sample of frozen cell-free supernatant obtained from *Pichia pastoris* NRRL Y-11430 by homogenization and centrifugation as described in Example I was cleared by centrifugation and adjusted to pH 6.5 with hydrochloric acid. The supernatant then was dialyzed for 5.5 hours against distilled water (supernatant/water volume ratio of 1/10). The resulting solid crystalline alcohol oxidase was recovered by decanting the dialysis supernatant from the solid enzyme. The catalase activity, using the method of R. F. Beers and I. W. Sizer, *J. Biol. Chem., 195,* 133 (1952), of the starting cell-free supernatant was 6973 U/ml while the catalase activity of the dialysis supernatant was 6697 U/ml. Therefore, over 90% of the initial catalase activity in the cell-free supernatant remains in the dialysis supernatant after conclusion of the dialysis.

Example XII

The reactivity of the alcohol oxidase from *Pichia pastoris* towards various substrates was determined using the previously described assay Procedure A. The fermentation effluent from the aerobic fermentation of *Pichia pastoris* NRRL Y-11430 in a manner similar to the fermentation described before Example I was homogenized and centrifuged. The cell-free supernatant was fractionally precipitated with ammonium sulfate to yield a partially purified alcohol oxidase. Assay Procedure A was used with the appropriate substrate being substituted for methanol.

The results in terms of relative reactivity normalized with methanol set equal to 100 are listed below.

| Substrate | Relative reactivity |
|---|---|
| Methanol | 100 |
| Ethanol | 100 |
| 1-Propanol | 73 |
| 2-Propanol | 4 |
| 1-Butanol | 45 |
| 2-Methyl-1-propanol | 9 |
| 1-Pentanol | 5 |

These results indicate that this partially purified alcohol oxidase from *Pichia pastoris* is reactive towards the lower, straight chain, primary alcohols.

Example XIII

A run was carried out to determine the influence of the enzyme concentration on the formation of pure, crystalline alcohol oxidase during dialysis according to the alcohol oxidase purification procedure of the present invention. An aerobic fermentation of *Pichia pastoris* NRRL Y-11430 was carried out at a cell density of about 150 g (dry weight) per liter. The fermenter effluent was homogenized and centrifuged to yield a cell-free supernatant. A series of samples was prepared from the cell-free supernatant by a series of dilutions with water. Five of the samples (2 ml each) were dialyzed against 100 ml of 0.01 M phosphate buffer (pH 6.5) for 4—5 hours. Sample 6 was a duplicate of sample 5 and was not dialyzed. At the conclusion of the dialyses, the liquids in the 5 dialysis bags and the undialyzed sample were assayed for enzyme activity by assay Procedure A. Where precipitates were formed during the dialysis, the solid was isolated by decanting the liquid and was dissolved in 2 ml of a phosphate buffer for assay by Procedure A. The results are presented below.

| Sample | Dilution | Effective cell density[(a)], g/l | Activity before dialysis[(b)], U/ml | Activity of super-natant[(c)], U/ml | Activity of precipi-tate[(d)], U/ml |
|---|---|---|---|---|---|
| 1 | Undiluted | 150 | 200 | 35.5 | 165.6 |
| 2 | 2× | 75 | 100 | 56.8 | 45.1 |
| 3 | 4× | 37.5 | 50 | 53.3 | (e) |
| 4 | 8× | 18.75 | 25 | 25.9 | (e) |
| 5 | 16× | 9.4 | 11 | 11.3 | (e) |
| 6 | 16×[(f)] | 9.4 | 10.1 | — | — |

[(a)] Cell density of the fermenter effluent represented by the diluted sample in g (dry weight)/l of broth.

[(b)] Estimated activity before dialysis is calculated from the activity of sample 5 after dialysis assuming no change in activity during dialysis and from the activity of undialyzed sample 6.

[(c)] Activity of liquid in dialysis bag after dialysis.

[(d)] Determined by dissolving solid precipitate in 2 ml of 0.5 M phosphate buffer (pH 7.5) for the assay.

[(e)] No precipitate formed during dialysis.

[(f)] This sample was not dialyzed.

Precipitates were formed during the dialysis of samples 1 and 2 which were the undiluted supernatant (150 g-dry/l cell density) and a two-fold dilution (representing an effective cell density of about 75 g-dry/l cell density) respectively. Precipitates were not formed in samples at the higher dilutions, which represent fermenter effluents from fermentations at effective cell densities of about 37.5 g (dry weight)/liter of broth and below. This suggests that fermenter effluents from fermentations at cell densities below about 40 g (dry weight)/liter of broth are not suitable for crystalline alcohol oxidase recovery. However, the fermenter effluents from such lower cell density fermentations can be concentrated using techniques such as ultrafiltrations, salt or solvent precipitation, and the like to provide materials suitable for the isolation of alcohol oxidase by my dialysis process.

**Claims**

1. A method for producing alcohol oxidase from aqueous fluids containing a suspension of cells of a methanol utilizing microorganism of genus Pichia characterized by preparing an aqueous fluid having a cell density effective for crystallization of alcohol oxidase in a recovery range solution having a molar ionic strength in the range of 0.05 M to 0.01 M, homogenizing said cell-containing fluid to produce a homogenate, and then removing suspended solids from said homogenate, thereby producing a solution containing soluble alcohol oxidase.

2. The method of claim 1 characterized by dialyzing the thus prepared solution across a membrane impermeable to the alcohol oxidase but permeable to water and buffer molecules, if any, against a dialysis medium to achieve said recovery range solution on an enzyme side of said membrane thereby resulting in crystalline alcohol oxidase, and separating the thus produced crystalline alcohol oxidase from the dialysis medium.

3. The method of claim 1 or 2 characterized by said cell-containing aqueous fluid having been prepared by culturing said genus Pichia microorganism under methanol-limiting conditions.

4. The method of claim 1 or 2 characterized in that said microorganism is selected from Pichia pastoris, Pichia pinus, Pichia trehalophila, and Pichia molischiana.

5. The method of claim 4 characterized in that said microorganism is selected from the strains Pichia pastoris NRRL Y-11430 and Pichia pastoris NRRL Y-11431.

6. The method of claim 1 or 2 characterized by using as said cell-containing aqueous fluid a fermenter effluent having been obtained by aerobic fermentation.

7. The method of claim 6 characterized by said fermenter effluent having been subjected to centrifugation to separate the cells from said fermenter effluent and said cells having been resuspended in an aqueous medium to form said cell-containing aqueous fluid.

8. The method of claim 6 characterized by said fermenter effluent having been subjected to filtration to separate the cells from said fermenter effluent and said cells having been resuspended in an aqueous medium to form said cell-containing aqueous fluid.

9. The method of claim 6 characterized in that said cells have a density in said cell-containing aqueous fluid of greater than 75 grams on a dry weight basis per liter of fluid.

10. The method of claim 6 characterized by removing suspended solids from the homogenate by centrifugation.

11. The method of claim 6 characterized by said recovery range solution is achieved at equilibration in the dialysis process.

12. The method of claim 11 characterized in that said recovery range solution has a molar ionic strength of 0.02 M.

13. The method of claim 6 characterized in that said recovery range solution is achieved prior to equilibration in the dialysis process.

14. The method of claim 13 characterized by separating a thus produced crystalline alcohol oxidase from the dialysis medium when said recovery range solution is about 0.02 M ionic strength.

15. The method of claim 6 characterized by separating the thus produced crystalline alcohol oxidase from the dialysis medium as a moist slurry followed by lyophilization.

16. An enzyme preparation from Pichia pastoris catalyzing the following reaction:

$$RCH_2OH+O_2 \rightleftharpoons RCHO+H_2O_2$$

wherein R is hydrogen, methyl, ethyl or propyl, the enzyme being characterized by its capacity to crystallize as an electrophoretically homogeneous crystalline oxidase from an aqueous solution having an effective cell density of at least 75 g (on a dry weight basis) per liter of fluid under dialysis of a pH in the range of 5.75 to 6.75 at a recovery range solution ionic strength from 0.05 to 0.01 molar.

17. The enzyme preparation of claim 16 characterized by containing an alcohol oxidase obtained from Pichia pastoris according to any of claims 1 to 15.

18. The enzyme preparation of claim 16 or 17 characterized by having a reactivity in the presence of n-propanol or n-butanol which is less than 75% of the reactivity of the enzyme in the presence of methanol.

19. The enzyme preparation of claim 18 characterized by having a reactivity in the presence of 2-propanol, 2-methyl-1-propanol or 1-pentanol which is less than 10% of the reactivity of the enzyme in the presence of methanol.

20. The enzyme preparation of claim 19 characterized by having a reactivity relative to methanol in the presence of the following compounds as set forth:

| | |
|---|---|
| Methanol | 100% |
| Ethanol | 100% |
| n-Propanol | 73% |
| n-Butanol | 45% |
| 2-Propanol | 4% |
| 2-Methyl-1-propanol | 9% |
| 1-Pentanol | 5% |

21. The enzyme preparation of claim 19 characterized in that said enzyme preparation comprises crystalline, electrophoretically homogeneous enzyme.

22. The enzyme preparation of claim 20 characterized in that said enzyme preparation is essentially lacking in catalase activity.

23. The enzyme preparation of claim 19 characterized in that said enzyme preparation comprises powdered enzyme produced by lyophilization of a crystalline enzyme containing fluid.

24. A method for determining the concentration of a compound in a fluid sample containing an alcohol selected from methanol, ethanol, n-propanol and n-butanol characterized by preparing said fluid sample to have conditions compatible with enzymatic activity, adding an alcohol oxidase preparation from a methanol utilizing microorganism of Pichia pastoris to said sample, and measuring a change of concentration in said sample of a compound selected from oxygen, hydrogen peroxide, and product aldehydes.

25. The method of claim 24 characterized in that said conditions compatible with enzymatic activity include said fluid sample having a pH in the range of 6 to 9 and having a temperature in the range of 25 to 45°C, and said alcohol oxidase preparation is electrophoretically homogeneous alcohol oxidase.

26.The method of claim 25 characterized in that said pH is about pH 8, and said temperature is about 45°C.

27. The method of claim 25 characterized in that said alcohol oxidase is immobilized on the tip of a dissolved oxygen electrode, said tip of said electrode is inserted into said sample, and a change of concentration of a compound selected from oxygen and hydrogen peroxide is measured polarographically.

28. The method of claim 27 characterized in that said fluid sample comprises a biological fluid, and said method is used to determine the concentration of a short chain alcohol.

29. The method of claim 28 characterized in that said biological fluid is blood, and said short chain alcohol is ethanol.

30. The alcohol oxidase from Pichia pastoris.

31. The concentrated alcohol oxidase from Pichia pastoris.

32. The crystalline alcohol oxidase from Pichia pastoris.

33. An enzyme electrode characterized by a polarographic dissolved oxygen electrode and an alcohol oxidase preparation according to any of claim 16 to 23 immobilized on the tip of said polarographic dissolved oxygen electrode.

34. The electrode of claim 33 characterized in that the enzyme preparation is blended with supporting material to form a paste which is held as a thin film on the electrode tip by a membrane permeable to the compound whose concentration is to be determined but impermeable to the enzyme preparation itself.

35. The electrode of claim 34 characterized in that the supporting material is a polysaccharide ion exchange resin and the membrane is a cellulose acetate film.

**Patentansprüche**

1. Verfahren zur Herstellung von Alkohol-Oxidase aus wäßrigen, eine Suspension von Zellen eines Methanol-verarbeitenden Mikroorganismus' der Gattung Pichia enthaltenden Fluiden, gekennzeichnet durch die Herstellung eines wäßrigen Fluids mit einer für die Kristallisation von Alkohol-Oxidase in einer Lösung im Rückgewinnungsbereich mit einer molaren Ionenstärke im Bereich von 0,05 bis 0,01 molar wirksammen Zelldichte, Homogenisierung des zellhaltigen Fluids unter Erzeugung eines Homogenisats und anschließende Entfernung suspendierter Feststoffe aus dem Homogenisat unter Bildung einer Lösung, die lösliche Alkohol-Oxidase enthält.

2. Verfahren nach Anspruch 1, gekennzeichnet durch Dialysieren der so hergestellten Lösung durch eine für die Alkohol-Oxidase undurchlässige, jedoch für Wasser und Puffermoleküle, wenn vorhanden, durchlässige Membran, gegen ein Dialysemedium unter Erzeugung der Lösung im Rückgewinnungsbereich an einer Enzymseite der Membran, wobei kristalline Alkohol-Oxidase anfällt, und Abtrennung der so hergestellten kristallinen Alkohol-Oxidase vom Dialysemedium.

3. Verfahren nach Anspruch 1 oder 2, dadurch gekennzeichnet, daß das zellhaltige wäßrige Fluid durch Züchtung des Mikroorganismus' der Gattung Pichia unter methanolbegrenzenden Bedingungen hergestellt worden ist.

4. Verfahren nach Anspruch 1 oder 2, dadurch gekennzeichnet, daß der Mikroorganismus aus Pichia pastoris, Pichia pinus, Pichia trehalophila und Pichia molischiana ausgewählt ist.

5. Verfahren nach Anspruch 4, dadurch gekennzeichnet, daß der Mikroorganismus aus den Stämmen Pichia pastoris NRRL Y-11430 und Pichia pastoris NRRL Y-11431 ausgewählt ist.

6. Verfahren nach Anspruch 1 oder 2, dadurch gekennzeichnet, daß als zellhaltiges wäßriges Fluid ein Fermenter-Austrag verwendet wird, der durch aerobe Fermentation erhalten worden ist.

7. Verfahren nach Anspruch 6, dadurch gekennzeichnet, daß der Fermenter-Austrag einer Zentrifugation unter Abtrennung der Zellen vom Fermenter-Austrag unterworfen worden ist, und die Zellen in einem wäßrigen Medium unter Bildung des zellhaltigen wäßrigen Fluids resuspendiert worden sind.

8. Verfahren nach Anspruch 6, dadurch gekennzeichnet, daß der Fermenter-Austrag einer Filtration unter Abtrennung der Zellen aus dem Fermenter-Austrag unterworfen worden ist, und die Zellen unter Bildung des zellhaltigen wäßrigen Fluids in einem wäßrigen Medium resuspendiert worden sind.

9. Verfahren nach Anspruch 6, dadurch gekennzeichnet, daß die Zellen eine Dichte im zellhaltigen wäßrigen Fluid von mehr als 75 Gramm, bezogen auf Trockensubstanz, pro Liter des Fluids aufweisen.

10. Verfahren nach Anspruch 6, dadurch gekennzeichnet, daß die suspendierten Feststoffe vom Homogenisat durch Zentrifugation entfernt werden.

11. Verfahren nach Anspruch 6, dadurch gekennzeichnet, daß die Lösung im Rückgewinnungsbereich im Dialyseprozess-Gleichgewichtszustand gewonnen wird.

12. Verfahren nach Anspruch 11, dadurch gekennzeichnet, daß die Lösung im Rückgewinnungsbereich eine molare Ionenstärke von 0,02 molar aufweist.

13. Verfahren nach Anspruch 6, dadurch gekennzeichnet, daß die Lösung im Rückgewinnungsbereich von Eintritt des Gleichgewichtszustandes im Dialyseprozess gewonnen wird.

14. Verfahren nach Anspruch 13, dadurch gekennzeichnet, daß eine so hergestellte kristalline Alkohol-Oxidase vom Dialysemedium abgetrennt wird, wenn die Lösung im Rückgewinnungsbereich eine Ionenstärke von etwa 0,02 molar aufweist.

15. Verfahren nach Anspruch 6, dadurch gekennzeichnet, daß die so hergestellte kristalline Alkohol-Oxidase vom Dialysemedium als feuchte Aufschlämmung, gefolgt von einer Gefriertrocknung, abgetrennt wird.

16. Enzympräparat aus Pichia pastoris, das folgende Reaktion katalysiert:

$$RCH_2OH+O_2 \rightleftharpoons RCHO+H_2O_2$$

wobei R ein Wasserstoffatom, eine Methyl-, Ethyl- oder Propylgruppe ist, und das Enzym durch seine Fähigkeit gekennzeichnet ist, als elektrophoretisch homogene kristalline Oxidase aus einer wäßrigen Lösung mit einer effektiven Zelldichte von mindestens 75 g (bezogen auf Trockengewicht) pro Liter Fluid bei der Dialyse bei einem pH-Wert im Bereich von 5,75 bis 6,75 und einer Ionenstärke der Lösung im Rückgewinnungsbereich von 0,05 bis 0,01 molar zu kristallisieren.

17. Enzympräparat nach Anspruch 16, gekennzeichnet durch den Gehalt an einer Alkohol-Oxidase, die aus Pichia pastoris gemäß einem der Ansprüche 1 bis erhalten worden ist.

18. Enzympräparat nach Anspruch 16 oder 17, gekennzeichnet durch eine Reaktivität in Gegenwart von n-Propanol oder n-Butanol, die weniger als 75% der Reaktivität des Enzyms in Gegenwart von Methanol beträgt.

19. Enzympräparat nach Anspruch 18, gekennzeichnet durch eine Reaktivität in Gegenwart von 2-Propanol, 2-Methyl-1-propanol oder 1-Pentanol, die weniger als 10% der Reaktivität des Enzyme in Gegenwart von Methanol beträgt.

20. Enzympräparat nach Anspruch 19, gekennzeichnet durch eine Reaktivität, relativ zu Methanol, in Gegenwart der folgenden Verbindungen, wie folgt:

| | |
|---|---|
| Methanol | 100% |
| Ethanol | 100% |
| n-Propanol | 73% |
| n-Butanol | 45% |
| 2-Propanol | 4% |
| 2-Methyl-1-propanol | 9% |
| 1-Pentanol | 5% |

21. Enzympräparat nach Anspruch 19, gekennzeichnet durch den Gehalt an kristallinem, elektrophoretisch homogenem Enzym.

22. Enzympräparat nach Anspruch 20, dadurch gekennzeichnet, daß praktisch keine Katalase-Aktivität vorliegt.

23. Enzympräparat nach Anspruch 19, gekennzeichnet durch den Gehalt an pulverförmigem Enzym, das durch Gefriertrocknung eines Fluids, welches ein kristallines Enzym enthält, hergestellt worden ist.

24. Verfahren zur Bestimmung der Konzentration einer Verbindung in einer Fluid-Probe, die einen Alkohol, ausgewählt aus Methanol, Ethanol, n-Propanol und n-Butanol enthält, dadurch gekennzeichnet, daß man die Fluid-Probe derart herstellt, daß sie die Voraussetzungen für eine Verträglichkeit mit enzymatischer Aktivität erfüllt, ein Alkohol-Oxidase Präparat aus einem methanolverarbeitendem Mikroorganismus der Art Pichia pastoris zur Probe gibt, und eine Konzentrationsänderung eines stoffes, ausgewählt aus Sauerstoff, wasserstoffperoxid und Produktaldehyde, in der Probe mißt.

25. Verfahren nach Anspruch 24, dadurch gekennzeichnet, daß die Verträglichkeits-Voraussetzungen für die enzymmatische Aktivität derart sind, daß die Fluid-Probe einen pH-Wert im Bereich von 6 bis 9 und eine Temperatur im Bereich von 25 bis 45°C aufweist, und das Alkohol-Oxidase-Präparat elektrophoretisch homogene Alkohol-Oxidase ist.

26. Verfahren nach Anspruch 25, dadurch gekennzeichnet, daß der pH-Wert etwa 8 und die Temperatur etwa 45°C beträgt.

27. Verfahren nach Anspruch 25, dadurch gekennzeichnet, daß die Alkohol-Oxidase auf der Spitze einer gelösten Sauerstoffelektrode immobilisiert ist, die Spitze der Elektrode in die Probe eingetaucht wird und eine Konzentrationsänderung eines Stoffes ausgewählt aus Sauerstoff und Wasserstoffperoxid polarographisch gemessen wird.

28. Verfahren nach Anspruch 27, dadurch gekennzeichnet, daß die Fluid-Probe ein biologisches Fluid enthält, und das Verfahren zur Bestimmung der Konzentration eines kurzkettigen Alkohols verwendet wird.

29. Verfahren nach Anspruch 28, dadurch gekennzeichnet, daß das biologische Fluid Blut ist, und der kurzkettige Alkohol Ethanol.

30. Alkohol-Oxidase aus Pichia pastoris.

31. Konzentrierte Alkohol-Oxidase aus Pichia pastoris.

32. Kristalline Alkohol-Oxidase aus Pichia pastoris.

33. Enzymelektrode, gekennzeichnet durch eine polarographisch gelöste Sauerstoffelektrode und ein Alkohol-Oxidase-Präparat gemäß einem der Ansprüche 16 bis 23, das auf der Spitze der polarographisch gelösten Sauerstoffelektrode immobilisiert ist.

34. Elektrode nach Anspruch 33, dadurch gekennzeichnet, daß das Enzympräparat mit Trägermaterial unter Bildung einer Masse vermischt ist, die als dünner Film auf der Elektrodenspitze durch eine Membran

gehalten wird, welche für die Verbindung, deren Konzentration bestimmt werden soll, durchlässig, aber undurchlässig für das Enzympräparat selbst ist.

35. Elektrode nach Anspruch 34, dadurch gekennzeichnet, daß das Trägermaterial ein Polysaccharid-Ionenaustauscherharz ist, und die Membran ein Celluloseacetatfilm ist.

**Revendications**

1. Procédé de production d'alcool-oxydase à partir de fluidesaqueux contenant une suspension de cellules d'un microorganisme utilisant du méthanol du genre Pichia, caractérisé en ce qu'on prépare un fluide aqueux ayant une densité cellulaire efficace pour la cristallisation d'une alcool-oxydase dans une solution dans l'intervalle de récupération ayant une force ionique molaire dans l'intervalle de 0,05 M à 0,01 M, en ce qu'on homogénéise ce fluide contenant les cellules pour produire un homogénéisat, puis en ce qu'on élimine les solides en suspension de cet homogénéisat, produisant ainsi une solution contenant de l'alcool-oxydase soluble.

2. Procédé de la revendication 1, caractérisé en ce qu'on dialyse la solution ainsi préparée à travers une membrane imperméable à l'alcool-oxydase, mais perméable à l'eau et aux molécules de tampon, s'il y en a, contre un milieu de dialyse pour réaliser cette solution dans l'intervalle de récupération du côté enzymes de cette membrane, ce qui conduit à une alcool-oxydase cristalline, et en ce qu'on sépare l'alcool-oxydase cristalline ainsi produite du milieu de dialyse.

3. Procédé suivant les revendications 1 ou 2, caractérisé en ce que ce fluide aqueux contenant les cellules a été préparé en cultivant ce microorganisme du genre Pichia dans des conditions limitant le méthanol.

4. Procédé suivant les revendications 1 ou 2, caractérisé en ce que ce microorganisme est choisi parmi Pichia pastoris, Pichia pinus, Pichia trehalophila, et Pichia molischiana.

5. Procédé de la revendication 4, caractérisé en ce que ce microorganisme est choisi parmi les souches Pichia pastoris NRRL Y-11430 et Pichia pastoris NRRL Y-11431.

6. Procédé suivant les revendications 1 ou 2, caractérisé en ce qu'on utilise comme ce fluide aqueux contenant les cellules un effluent de fermenteur qui a été obtenu par fermentation aérobie.

7. Procédé de la revendication 6, caractérisé en ce que cet effluent de fermenteur a été soumis à une centrifugation pour séparer les cellules de cet effluent de fermenteur et en ce que ces cellules ont été remises en suspension dans un milieu aqueux pour former ce fluide aqueux contenant des cellules.

8. Procédé de la revendication 6, caractérisé en ce que cet effluent de fermenteur a été soumis à une filtration pour séparer les cellules de cet effluent de fermenteur et en ce que ces cellules ont été remises en suspension dans un milieu aqueux pour former ce fluide aqueux contenant des cellules.

9. Procédé de la revendication 6, caractérisé en ce que ces cellules ont une densité dans ce fluide aqueux contenant les cellules supérieure à 75 grammes sur la base des poids secs par litre de fluide.

10. Procédé de la revendication 6, caractérisé en ce qu'on élimine les solides en suspension de l'homogénéisat par centrifugation.

11. Procédé de la revendication 6, caractérisé en ce que cette solution dans l'intervalle de récupération est réalisée à l'équilibrage dans le procédé de dialyse.

12. Procédé de la revendication 11, caractérisé en ce que cette solution dans l'intervalle de récupération a une force ionique molaire de 0,02 M.

13. Procédé de la revendication 6, caractérisé en ce que cette solution dans l'intervalle de récupération est réalisée avant l'équilibrage dans le procédé de dialyse.

14. Procédé de la revendication 13, caractérisé en ce qu'on sépare une alcool-oxydase cristalline ainsi produite du milieu de dialyse lorsque cette solution dans l'intervalle de récupération a une force ionique d'environ 0,02 M.

15. Procédé de la revendication 6, caractérisé en ce qu'on sépare l'alcool-oxydase cristalline ainsi produite du milieu de dialyse sous la forme d'une bouillie humide puis en ce qu'on la lyophilise.

16. Préparation d'enzyme de Pichia pastoris catalysant la réaction suivante:

$$RCH_2OH+O_2 \rightleftharpoons RCHO+H_2O_2$$

dans laquelle R est l'hydrogène, un groupe méthyle, éthyle ou propyle, l'enzyme étant caractérisée par sa capacité de cristalliser sous la forme d'une oxydase cristalline électrophorétiquement homogène à partir d'une solution aqueuse ayant une densité effective en cellules d'au moins 75 g (sur la base des poids secs) par litre de fluide sous dialyse, à un pH d'un intervalle de 5,75 à 6,75, pour une force ionique de la solution dans l'intervalle de récupération de 0,05 à 0,01 molaire.

17. Préparation d'enzyme de la revendication 16, caractérisée en ce qu'elle contient une alcool-oxydase obtenue à partir de Pichia pastoris conformément à l'une quelconque des revendications 1 à 15.

18. Préparation d'enzyme des revendications 16 ou 17, caractérisée en ce qu'elle a une réactivité en présence de n-propanol ou de n-butanol qui est inférieure à 75% de la réactivité de l'enzyme en présence de méthanol.

19. Préparation d'enzyme de la revendication 18, caractérisée en ce qu'elle a une réactivité en présence

de 2-propanol, de 2-méthyl-1-propanol ou de 1-pentanol, qui est inférieure à 10% de la réactivité de l'enzyme en présence de méthanol.

20. Préparation d'enzyme de la revendication 19, caractérisée en ce qu'elle a une réactivité par rapport au méthanol en présence des composés suivants, qui est celle indiqués ci-dessous:

| | |
|---|---|
| Méthanol | 100% |
| Ethanol | 100% |
| n-propanol | 73% |
| n-butanol | 45% |
| 2-propanol | 4% |
| 2-méthyl-1-propanol | 9% |
| 1-pentanol | 5% |

21. Préparation d'enzyme de la revendication 19, caractérisée en ce que cette préparation d'enzyme comprend une enzyme cristalline, électrophorétiquement homogène.

22. Préparation d'enzyme de la revendication 20, caractérisée en ce que cette préparation d'enzyme manque pratiquement d'activité de catalase.

23. Préparation d'enzyme de la revendication 19, caractérisée en ce que cette préparation d'enzyme comprend une enzyme pulvérisée produite par lyophilisation d'un fluide contenant une enzyme cristalline.

24. Procédé pour la détermination de la concentration d'un composé dans un échantillon de fluide contenant un alcool choisi parmi le méthanol, l'éthanol, le n-propanol et le n-butanol, caractérisé en ce qu'on prépare cet échantillon de fluide de façon à ce qu'il ait des conditions compatibles avec l'activité enzymatique, en ce qu'on ajoute une préparation d'alcool-oxydase provenant d'un microorganisme utilisant le méthanol de Pichia pastoris à cet échantillon, et en ce qu'on mesure la variation de concentration dans cet échantillon d'un composé choisi parmi l'oxygène, le peroxyde d'hydrogène et des aldéhydes.

25. Procédé de la revendication 24, caractérisé en ce que ces conditions de l'échantillon de fluide compatibles avec l'activité enzymatique comprennent un pH d'un intervalle de 6 à 9 et une température dans l'intervalle de 25 à 45°C, et en ce que cette préparation d'alcool-oxydase est de l'alcool oxydase électrophorétiquement homogène.

26. Procédé de la revendication 25, caractérisé en ce que ce pH est d'environ 8 et en ce que cette température est d'environ 45°C.

27. Procédé de la revendication 25, caractérisé en ce que cette alcool-oxydase est immobilisée sur la pointe d'une électrode à oxygène dissous, cette pointe de cette électrode est insérée dans cet échantillon, et une variation de concentration d'un composé choisi parmi l'oxygène et le peroxyde d'hydrogène est mesurée polarographiquement.

28. Procédé de la revendication 27, caractérisé en ce que cet échantillon de fluide comprend un fluide biologique, et en ce que ce procédé est utilisé pour déterminer la concentration d'un alcool à chaîne courte.

29. Procédé de la revendication 28, caractérisé en ce que ce fluide biologique est le sang, et en ce que cet alcool à chaîne courte est l'éthanol.

30. Alcool-oxydase de Pichia pastoris.

31. Alcool-oxydase concentrée de Pichia pastoris.

32. Alcool-oxydase cristalline de Pichia pastoris.

33. Electrode à enzyme, caractérisée par une électrode polarographique à oxygène dissous et une préparation d'alcool-oxydase suivant l'une quelconque des revendications 16 à 23 immobilisée sur la pointe de cette électrode polarographique à oxygène dissous.

34. Electrode de la revendication 33, caractérisée en ce que la préparation d'enzyme est mélangée avec une matière de support pour former une pâte qui est maintenue sous la forme d'une pellicule fine sur la pointe d'électrode par une membrane perméable au composé, dont la concentration doit être déterminée, mais imperméable à la préparation d'enzyme elle-même.

35. Electrode de la revendication 34, caractérisée en ce que cette matière de support est une résine échangeuse d'ions de polysaccharide et en ce que la membrane est une pellicule d'acétate de cellulose.